(19) Europäisches Patentamt
European Patent Office
Office européen des brevets

(11) Veröffentlichungsnummer: **0 199 330**
**B1**

(12) **EUROPÄISCHE PATENTSCHRIFT**

(45) Veröffentlichungstag der Patentschrift: **19.12.90**

(21) Anmeldenummer: **86105499.7**

(22) Anmeldetag: **21.04.86**

(51) Int. Cl.⁵: **C 07 C 47/42,** C 07 C 49/543, C 07 C 69/75, C 07 C 33/14, C 07 C 45/69, C 11 B 9/00, A 61 K 7/46

(54) **Trimethylcyclohexen-Derivate, ihre Herstellung und deren Verwendung als Duftstoffe.**

(30) Priorität: **23.04.85 DE 3514665**

(43) Veröffentlichungstag der Anmeldung:
**29.10.86 Patentblatt 86/44**

(45) Bekanntmachung des Hinweises auf die Patenterteilung:
**19.12.90 Patentblatt 90/51**

(84) Benannte Vertragsstaaten:
**CH DE FR GB LI NL**

(56) Entgegenhaltungen:
**FR-A-2 183 075**
**GB-A-2 005 671**
**US-A-2 794 812**
**US-A-3 220 977**
**US-A-4 038 326**

(73) Patentinhaber: **Consortium für elektrochemische Industrie GmbH**
**Zielstattstrasse 20**
**D-8000 München 70 (DE)**

(72) Erfinder: **Gebauer, Helmut, Dr. Dipl.-Chem.**
**Schaffhauser Strasse 18**
**D-8000 München 71 (DE)**
Erfinder: **Mehlin, Hans**
**Ammerseestrasse 8**
**D-8027 Neuried (DE)**
Erfinder: **Regiert, Marlies**
**Schraudolphstrasse 2a**
**D-8000 München 40 (DE)**

**EP 0 199 330 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft neue Verbindungen aus der Gruppe der 1,4,6-Trimethyl-cyclohex-3-en-derivate.

Gemäß S. Arcander, "Perfumes and Flavour Chemicals" (1969), sind bereits der 3,5,6-Trimethyl-cyclohex-3-en-1-carbaldehyd und der 2,4,6-Trimethyl-cyclohex-3-en-1-carbaldehyd bekannt. Das Geruchsbild der genannten Duftstoffe wird durch süße, blumige Noten bestimmt.

Es wurde nun gefunden, daß eine Auswahl von 1,4,6-Trimethylcyclohex-3-enen, die in 1-Stellung ein quartäres Kohlenstoffatom aufweisen, in einem völlig anderen Bereich des Duftspektrums einzuordnen sind, als die oben genannten, bekannten Trimethylcyclohexen-Derivate, nämlich in dem Bereich der würzigen und holzigen Noten. Insbesondere ist hervorzuheben, daß einige Vertreter der erfindungsgemäßen Verbindungen die wesentlichen Geruchsaspekte des natürlichen Patchouli-Öls abdecken.

Gegenstand der Erfindung sind Verbindungen der allgemeinen Formel

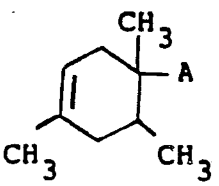

wobei A die

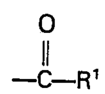

oder die

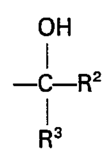 Gruppe

bedeutet und wobei

$R^1$ für Wasserstoff, Methyl, Ethyl, n-Propyl; iso-Propyl, Methoxy, Ethoxy, n-Propoxy und iso-Propoxy steht und $R^2$ und $R^3$ Wasserstoff, Methyl und Ethyl bedeuten.

Beispiele für erfindungsgemäße Verbindungen sind

1,4,6-Trimethyl-cyclohex-3-en-1-carbaldehyd
1,4,6-Trimethyl-cyclohex-3-en-1-carbonsäuremethylester
1,4,6-Trimethyl-cyclohex-3-en-1-carbonsäureethylester
1,4,6-Trimethyl-cyclohex-3-en-1-carbonsäurepropylester
1,4,6-Trimethyl-cyclohex-3-en-1-carbonsäure-iso-propylester
(1,4,6-Trimethyl-cyclohex-3-en-1-yl)-methanol
1,4,6-Trimethyl-cyclohex-3-en-1-yl)-ethan-1-ol
1,4,6-Trimethyl-cyclohex-3-en-1-yl)-propan-1-ol
2-(1,4,6-Trimethyl-cyclohex-3-en-1-yl)-propan-2-ol
2-(1,4,6-Trimethyl-cyclohex-3-en-1-yl)-butan-2-ol
3-(1,4,6-Trimethyl-cyclohex-3-en-1-yl)-pentan-3-ol
1-(1,4,6-Trimethyl-cyclohex-3-en-1-yl)-ethan-1-on
1-(1,4,6-Trimethyl-cyclohex-3-en-1-yl)-propan-1-on
1-(1,4,6-Trimethyl-cyclohex-3-en-1-yl)-butan-1-on
1-(1,4,6-Trimethyl-cyclohex-3-en-1-yl)-3-methyl-propan-1-on.

Der erfindungsgemäße 1,4,6-Trimethyl-cyclohex-3-en-1-carbaldehyd und die analogen erfindungsgemäßen Ketone sind direkt durch 1,4-Cycloaddition von Isopren mit Tiglinaldehyd bzw. mit den entsprechenden 2-Propenyl-alkyl-ketonen zugänglich.

Der erfindungsgemäße Carbaldehyd und die analogen Ketone sind direkt durch 1,4-Cycloaddition von Tiglinaldehyd bzw. den entsprechenden 2-Propenyl-alkyl-Ketonen mit Isopren zugänglich.

Die erfindungsgemäßen primären und sekundären Alkohole werden in an sich bekannter Weise durch Reduktion des Aldehyds bzw. durch Reduktion der entsprechenden Ketone gewonnen. Beispiele für geeignete Reduktionsmittel sind komplexe Hydride wie Natriumborhydrid, Lithium aluminiumhydrid oder Aluminiumisopropylat (nach Meerwein-Ponndorf).

Die erfindungsgemäßen tertiären Alkohole sind beispielsweise aus den entsprechenden Ketonen in an sich bekannter Weise durch Reaktion mit Grignard-Verbindungen wie Methyl- und Ethylmagnesiumhalogenid erhältlich.

Die erfindungsgemäßen Ester sind durch Oxidation des Carbaldehyds zur Säure (z.B. mit Chromschwefelsäure als Oxidationsmittel) und anschließende Veresterung der Säure, gegebenenfalls über die Zwischenstufe des Carbonsäurechlorids, zugänglich.

Das bevorzugte Verfahren zur Herstellung der erfindungsgemäßen Verbindungen ist dadurch gekennzeichnet, daß

a) Isopren und ein Dienophil der Formel

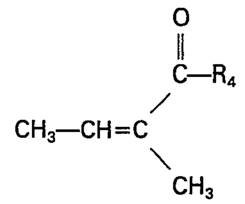

wobei $R_4$ Wasserstoff oder eine Alkylgruppe mit 1—3 Kohlenstoffatomen bedeutet, einer 1,4-Cycloaddition unterworfen werden und

b) gegebenenfalls das Reaktionsprodukt gemäß a)

b1) mittels Natriumborhydrid, Lithiumaluminiumhydrid oder Aluminiumisopropylat in den primären bzw. sekundären Alkohol,

b2) mittels Alkylmagnesiumhalogenid in den tertiären Alkohol, oder

b3) durch Oxidation zur Säure und anschließende Veresterung in den Ester, übergeführt wird.

Beispiele für Dienophile sind Tiglinaldehyd, 3-Methyl-pent-3-en-2-on, 4-Methyl-hex-4-en-3-on, 2,4-Dimethyl-hex-4-en-3-on und 5-Methyl-hept-5-en-4-on.

Unter 1,4-Cycloaddition wird im Rahmen der Erfindung die auch als Diels-Alder-Reaktion bekannte Addition einer Verbindung mit 2 in Konjugation stehenden Kohlenstoffdoppelbindungen an ein Olefin, dessen Kohlenstoffdoppelbindung in Konjugation mit einer Carbonylgruppe steht, unter Ausbildung eines Cyclohexenderivats, verstanden. Die verbleibende Kohlenstoffdoppelbindung wird dabei an jener Stelle gebildet, die ursprünglich das Bindeglied zwischen den konjugierten Doppelbindungen des Diolefins bildete.

Isopren (2-Methyl-buta-1,3-dien) und Dienophil werden zweckmäigerweise im molaren Verhältnis von 0,9 bis 1,3 zu 1, insbesondere 1,01 bis 1,3 zu 1 eingesetzt.

Die Reaktionstemperaturen betragen zumeist 15 bis 130°C. Es kann sowohl mit als auch ohne Katalysator gearbeitet werden. Beime Arbeiten ohne Katalysator wird der Temperaturbereich von 80 bis 130°C bevorzugt. Die Umsetzungen werden dabei zweckmäßigerweise in Druckapparaturen bei 5 bis 20 bar absolut durchgeführt.

Dabei entstehen neben den 1,4,6-Trimethyl-cyclohexen-derivaten auch in geringerem Umfang die analogen 1,3,6-Trimethylcyclohexen-derivate, die falls erwünscht, durch an sich bekannte Methoden, beispielsweise durch Chromatographie, abgetrennt werden können.

In Gegenwart von Lewissäuren wird die Bildung der 1,3,6-Trimethyl-cyclohexenspecies weitgehend zurückgedrängt. Zudem wird ein katalytischer Effekt erzielt, der es gestattet, die 1,4-Cycloaddition auch bereits beim Druck der umgebenden Atmosphäre und bei Temperaturen von 15 bis 80°C durchzuführen.

Die Lewissäuren werden bevorzugt in Mengen von 0,1 bis 10 mol%, bezogen auf die Menge umzusetzenden Dienophils eingesetzt. Beispiele für erfindungsgemäß als Katalysatoren einzusetzende Lewissäuren sind Aluminiumchlorid, Zinkchlorid, Eisen-III-chlorid und andere.

Beispielsweise wird so vorgegangen, daß Dienophil und Lewissäure vorgelegt werden und Isopren zudosiert wird. Die Reaktionstemperaturen betragen dabei anfänglich zweckmäßigerweise etwa 15 bis 40°C und werden bis zum Ende der Reaktion allmählich auf 60 bis 80°C gesteigert. Die Reaktion verläuft exotherm. Die Reaktionskontrolle kann beispielsweise über die Dosiergeschwindigkeit des Isoprens gesteuert werden.

In einer weiteren Verfahrensvariante wird der Katalysator in einem inerten Lösungsmittel wie z.B. Dibutylether vorgelegt und eine Mischung der Reaktanten aus Isopren und Dienophil zugetropft.

Die erfindungsemäßen Verbindungen finden Verwendung als Riechstoffe bzw. als Bestandteile von Riechstoffmischungen. Sie können einzeln oder im Gemisch und insbesondere auch im Gemisch mit den analogen 1,3,6-Trimethyl-cyclohex-3-en-derivaten eingesetzt werden. Die genannten 1,3,6-Trimethylcyclohexene geben eion den 1,4,6-Cyclohex-3-en-derivaten sehr ähnliches Geruchsbild ab.

Die erfindungsgemäßen Duftstoffe sind in dem Bereich holziger und würziger Noten einzuordnen. Neben frisch-krautigen, würzig-blumigen bis erdig-holzigen Geruchsnoten treten, bisweilen auch ausgeprägt Noten hervor, die an Ingwer, Calmus und Salbei erinnern. Sie weisen durchweg große Geruchsintensität auf.

Insbesondere bei 1-(1,4,6-Trimethyl-cyclohex-3-en-1-yl)-ethan-1-ol, bei 1-(1,4,6-Trimethyl-cyclohex-3-en-1-yl)-propan-1-on, bei (1,4,6-Trimethyl-cyclohex-3-en-1-yl)-methanol und, besonders hervorzuheben bei 1-(1,4,6-Trimethyl-cyclohex-3-en-1-yl)-1-methyl-ethan-1-ol sowie bei 1-(1,4,6-Trimethyl-cyclohex-3-en-1-yl)-1-methyl-propan-1-ol treten die wesentlichen Geruchsaspekte des natürlichen Patchouliöls hervor, das nur in begrenzter Menge und häufig sehr unterschiedlicher Qualität zur Verfügung steht. Die

geruchsbestimmenden Inhaltsstoffe des natürlichen Patchouliöls sind der trizyklische Sesquiterpenalkohol Patchoulol und das verwandte Norpatchoulenol, für die bisher keiner wirtschaftlich verwertbaren Synthesen bekannt sind. Mit den genannten erfindungsgemäßen Verbindungen stehen nunmehr vollsynthetisch zugängliche Duftstoffe zur Verfügung, die das natürliche Patchouliöl in Geruchskompositionen ersetzen können.

Die Erfindung wird nun anhand von Beispielen näher erläutert:

Beispiel 1

Herstellung von 1-(1,4,6-Trimethyl-cyclohex-3-en-1-yl)-ethan-1-on

In einem 1-Liter-4-Halskolben mit Rührer, Rückflußkühler, Tropftrichter und Innenthermometer wurden 2 mol (196 g) 3-Methyl-pent-3-en-2-on und 0,1 mol (13,3 g) wasserfreies Aluminiumchlorid vorgelegt. In diese Mischung wurden 3 mol (300 ml) Isopren su zudosiert, daß die Temperatur des Reaktionsgemisches auf 80°C stieg. Schließlich wurde abgekühlt, mit 100 ml 5 Gew.%iger Salzsäure versetzt und extrahiert. Danach wurde die organische Phase abgetrennt und ohne weitere Reinigung über eine 60 cm hohe Füllkörperkolonne fraktioniert destilliert. Die Ausbeute an Zielprodukt betrug 265 g, entsprechend 80% der Theorie.

Siedepunkt bei 16 mbar 93°C.

Geruch: frisch-minzig, grün-krautig, süß-balsamisch, an Rosmarin und Salbei erinnernd

Beispiel 2

Herstellung von 1-(1,4,6-Trimethyl-cyclohex-3-en-1-yl)-ethanol

In einem 1-Liter-3-Halskolben mit Magnetrührer, Füllkörperkolonne, Tropftrichter und Thermometer wurde eine Lösung von 0,33 mol (68 g) Aluminiumisopropylat in 600 ml Isopropanol zum Sieden erhitzt. In die siedende Lösung wurde innerhalb zwei Stunden 1 mol (166 g) 1-(1,4,6-Trimethyl-cyclo-hex-3-en-1-yl)-ethan-1-on (gemäß Beispiel 1) zugetropft und gleichzeitig das entstehende Isopropanol/Aceton-Gemisch abdestilliert. Nach 10 Stunden war die Reaktion beendet (nachdem im Destillat kein Aceton mehr nachzuweisen war). Danach wurde noch verbliebenes Isopropanol abgezogen und der Rückstand nach Abkühlen mit 175 ml 20 Gew.%iger Salzsäure versetzt. Es wurde extrahiert und die organische Phase säurefrei gewaschen. Nach fraktionierter Destillation wurden 151 g an Zielprodukt entsprechend 90% der Theorie erhalten.

Siedepunkt bei 0,2 mbar 65°C

Geruch: trocken, erdig-holzig-würzig, an Stroh und Calmus erinnernd

Beispiel 3

Herstellung von 2-(1,4,6-Trimethyl-cyclohex-3-en-1-yl)-propan-2-ol

a) in einem 500 ml-4-Halskolben mit Magentrührer, Rückflußkühler, Tropftrichter, Innenthermometer und Gaseinleitungsrohr wurden 0,5 mol (12 g) pulverförmiges Magnesiumvorgelegt und mit 20 ml trockenem Tetrahydrofuran und 1 ml Ethylbromid überschichtet. Nach dem Anspringen der Reaktion (Graufärbung und Wärmetönung) wurden bei 50 bis 60°C weitere 110 ml Tetrahydrofuran innerhalb einer Stunde zugetropft. Gleichzetig wurde Methylchlorid in das Gemisch eingeleitet bis alles Magnesium umgesetzt war.

b) danach wurden 0,4 mol (66,4 g) 1-(1,4,6-Trimethyl-cyclohex-3-en-1-yl)- ethanol (gemäß Beispiel 2) bei 70°C der Reaktionsmischung gemäß a) zudosiert. Danach wurde noch eine Stunde bei 70°C gerührt. Die Reaktionsmischung wurde nun abgekühlt, auf 300 g Eis gegossen und das ausgefallene Magnesiumsalz mit 30 ml Eisessig gelöst. Schließlich wurden die Phasen getrennt, die organische Phase mit Natriumhydrogencarbonat-Lösung neutralisiert, das Lösungsmittel abgezogen und der Rückstand fraktioniert destilliert. Es wurden 30 g entsprechend 40% der Theorie an Zielprodukt erhalten.

Siedebereich 65—67°C bei 0,2 mbar

Geruch: stark krautig, holzig, würzig, delikat-erdig-balsamisch, strahlend ausgeprägt Patchouli

Beispiel 4

Herstellung von 1-(1,4,6-Trimethyl-cyclohex-3-en-1-yl)-propan-1-on

In einem 250 ml-3-Halskolben mit Magnetrührer, Intensivkühler, Tropftrichter und Innenthermometer wurden unter kräftigem Rühren 0,025 mol (3,3 g) wasserfreies Aluminiumchlorid in 1 mol (112 g) 4-Methyl-hex-4-en-3-on gelöst. In diese Lösung wurden bei 40°C 1,5 mol (150 ml) Isopren zugetropft. Nach beendeter Zugabe wurde das Reaktionsgemisch noch 1 Stunde auf 80°C erwärmt. Nach dem Abkühlen wurde die Mischung mit 50 ml 5 Gew.- %iger Salzsäure versetzt, die Phasen getrennt und die organische Phase fraktioniert destilliert. Es wurden 144 g, entsprechend 80% der Theorie an Zielprodukt erhalten.

Siedepunkt bei 0,2 mbar 62°C

Geruch: frish krautig, leicht minzig und süß, balsamisch-holzig wie Rosmarin

Beispiel 5

Herstellung von 1-(1,4,6-Trimethyl-cyclohex-3-en-1-yl)-propan-1-ol

In einem 250 ml-3-Halskolben mit Magnetrührer, Intensivkühler, Tropftrichter und Innenthermometer wurde eine Lösung von 0,075 mol (2,85 g) Lithiumaluminiumhydrid in 150 ml absolutem Diethylether vorgelegt. In diese Lösung wurden unter Kühlung 0,2 mol (36 g) 1-(1,4,6-Trimethyl-cyclohex-3-en-1-yl)-propan-1-on (gemäß Beispiel 4) zugetropft. Danach wurde eine Stunde auf Rückfluß erhitzt, anschließend abgekühlt und das Reaktionsgemisch bis zur Beendigung der Wasserstoffentwicklung mit Wasser versetzt. Danach wurde mit soviel 2 normaler Salzsäure versetzt, daß der entstandene Niederschlag gelöst werden konnte. Schließlich wurden die Phasen getrennt, die organische Phase mit Natriumsulfat getrocknet und das Lösungstmittel abgezogen. Nach fraktionierter Destillation über eine 30 cm Vigreux-Kolonne wurde das Zielprodukt in praktisch quantitativer Ausbeute erhalten.

Siedepunkt: 65°C bei 0,15 mbar

Geruch: agrumig-frisch mit erdig-holzigen Aspekten

Beispiel 6

Herstellung von 2-(1,4,6-Trimethyl-cyclohex-3-en-1-yl)-butan-2-ol

a) es wurde nach der Arbeitsweise gemäß Beispiel 3a aus 12 g Magnesiumpulver die entsprechende Grignard-Verbindung hergestellt.

b) es wurden analog 3b 0,4 mol (72 g) 1-(1,4,6-Trimethyl-cyclohex-3-en-1-yl)-propan-1-on (gemäß Beispiel 4) mit der Grignard-Verbindung (gemäß a) umgesetzt. Das Zielprodukt wurde in einer Ausbeute von 48 g entsprechend 62% der Theorie erhalten.

Sidepunkt: 80°C bei 0,2 mbar

Geruch: frisch-würzig, leicht minzig, holzig-balsamisch, strahlend, Patchouli- und Salbeicharakter

Beispiel 7

Herstellung von 1,4,6-Trimethyl-cyclohex-3-en-1-carbaldehyd

Es wurde in einem 2-Liter-4-Halskolben mit Rührer, Intensivkühler, Tropftrichter und Innenthermometer eine Lösung von 0,2 mol (26,2 g) wasserfreien Aluminiumchlorids in 4 mol (336 g) 2-Methyl-but-2-en-1-al vorgelegt. In diese Lösung wurden 6 mol (600 ml) Isopren bei 40°C zugetropft. Danach wurde noch eine Stunde auf 60°C erwärmt. Nach dem Abkühlen wurde mit 200 ml 5 Gew.%iger Salzsäure versetzt, die Phasen getrennt und die organische Phase über eine 60 cm Füllkörper-Kolonne fraktioniert destilliert. Es wurden 350 g entsprechend 58% der Theorie an Zielprodukt erhalten.

Siedepunkt: 75°C bei 16 mbar

Geruch: agrumig-frisch, grün, würzig-holzig, Ingwer-Aspekte

Beispiel 8

Herstellung von 1,4,6-Trimethyl-cyclohex-3-en-1-carbynol

Das Zielprodukt wurde analog der Arbeitsweise gemäß Beispiel 5 in praktisch quantitativer Ausbeute durch Reduktion von 1,4,6-Trimethyl-cyclohex-3-en-1-carbaldehyd (gemäß Beispiel 7) mit Lithimaluminiumhydrid erhalten.

Siedepunkt: 84°C bei 16 mbar

Geruch: stark minzig-süß, erdig

Beispiel 9

Herstellung von 1,4,6-Trimethyl-cyclohex-3-en-1-carbonsäuremethylester

In einem 500 ml-3-Halskolben mit Rückflußkühler und Rührer wurden 0,2 mol 1,4,6-Trimethyl-cyclohex-3-en-1-carbaldehyd (gemäß Beispiel 7) in 100 ml Diethylether vorgelegt. In diese Lösung wurde bei 25°C Chromschwefelsäure (0,07 mol Natriumdichromat 15 ml konzentrierte Schwefelsäure, 100 ml Wasser) unter Rühren getropft.

b) in einem 100 ml-3-Halskolben mit Magnetrührer, Rückflußkühler, Tropftrichter und Innenthermometer wurden 0,1 mol (16,8 g) 1,4,6-Trimethyl-cyclohex-3-en-1-carbonsäure (gemäß a) mit 0,15 mol (11 ml) Thionylchlorid versetzt und eine Stunde unter Rückfluß erhitzt. Danach wurde überschüssiges Thionylchlorid abdestilliert.

c) in einem 100 ml Becherglas mit Magnetrührer wurden 2 mol (8 ml) Methanol, 0,15 mol (12 ml) Pyridin und 50 ml Toluol vorgelegt und unter kräftigem Rühren das Rohprodukt gemäß b (1,4,6-Trimethyl-cyclohex-3-en-1-carbonsäurechlorid) zudosiert. Die Reaktionsmischung wurde noch eine Stunde bei Raumtemperatur gerührt und anschließend mit 25 ml Wasser und 5 ml konzentrierter Salzsäure versetzt. Danach wurden die Phasen getrennt, die organische Phase mit Natriumsulfat getrocknet und nach Abziehen des Lösungsmittels über Vigreux-Kolonne destilliert.

Die Ausbeute an Zielprodukt betrug 13,5 g entsprechend 75% der Theorie.

Siedepunkt: 91°C bei 12 mbar

Geruch: süß-krautig, minzig

Beispiel 10

Parfümbase mit Cologne-Charakter

| | a Gew.T. | b Gew.T. |
|---|---|---|
| Grapefruitöl | 60 | 60 |
| Orangenöl Florida | 90 | 90 |
| Cedernholzöl Texas | 60 | 60 |
| Linalool | 100 | 100 |
| Linalylacetat | 170 | 170 |
| α-Amylzimtaldehyd | 90 | 90 |
| Lavendelöl Bareme | 80 | 80 |
| Nelkenblätteröl | 30 | 30 |
| Litsea-Cubebaöl | 60 | 60 |
| Elemi | 30 | 30 |
| Jasminbase 4a (10% in Ethanol) | 50 | 50 |
| Nerol | 50 | 50 |
| Verbindung 1 | — | 130 |
| | 870 | 1000 |

Die Komposition a weist einen frisch-fruchtigen Duft mit betont agrumigen Nuancen auf. Etwas unvermittelt und ohne rechte Verbindung sind daneben krautige Aspekte erkannbar. Durch Zugabe von 13% 1-(1,4,6-Trimethyl-cyclohex-3-en-1-yl)-ethan-1-on (Komposition b) verbinden sich fruchtige und krautig-holzige Komponenten zu einem harmonischen Geruchsbild eines würzig-maskulinen Colognes.

Beispiel 11

Florale Chypre-Base

| | a Gew.T. | b Gew.T. |
|---|---|---|
| Bergamottöl, Reggio | 150 | 150 |
| Eichenmoss, Res. Luxol | 50 | 50 |
| alpha-iso-Methylionen | 150 | 150 |
| Geraniumöl Bourbon | 30 | 30 |
| Jasminbase 4a (10% in Ethanol) | 50 | 50 |
| Vetiverylacetat | 60 | 60 |
| Nelkenöl | 50 | 50 |
| Ylang-Ylang-Öl | 50 | 50 |
| Amylsalicylat | 60 | 60 |
| Benzylacetat | 70 | 70 |
| Linaylacetat | 100 | 100 |
| Keton-Moschus | 50 | 50 |
| Labdanum resinoid | 30 | 30 |
| Undecalacton (10% in Ethanol) | 50 | 50 |
| Verbindung 3 | — | 50 |
| | 950 | 1000 |

Die Zugabe von 5% 2-(1,4,6-Trimethyl-cyclohex-3-en-1-yl)-propan-2-ol zu dem klassischen Chypre-Thema *a* bewirkt eine kräftige Erhöhung der Strahlung und Eleganz. Trotz der Betonung der Eichenmoos- und Holzaspekte erscheint die Komposition insgesamt ausgewogener, reifer.

Beispiel 12

| Holz-Base | a | b |
|---|---|---|
| Bergamottöl, Reggio | 120 | 120 |
| Linalylacetat | 140 | 140 |
| Sandelholzöl, ostind. | 120 | 120 |
| p-tert.-Butylcyclohexyl-acetat | 100 | 100 |
| Cedrylacetat | 120 | 120 |
| Jasminbase 4a, 10% in Ethanol | 100 | 100 |
| Methylionen | 80 | 80 |
| Patchouliöl | 100 | 100 |
| Verbindung 3 | — | 120 |
| | 880 | 1000 |

Gibt man zur Holz-Komposition a 12% 2-(1,4,6-Trimethyl-cyclohex-3-en-1-yl)-propan-2-ol, so wird die Strahlkraft enorm erhöht. Das Geruchsbild von edlen Hölzern wird intensiviert. Zudem wirkt der neue Bestandteil fixierend und veleiht der Base eine warme, pudrige Fondnote.

## Patentansprüche

1. Verbindungen der allgemeinen Formel

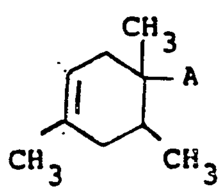

wobei A die

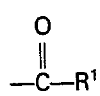 oder die 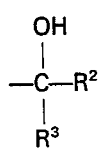 Gruppe

bedeutet und wobei

$R^1$ für Wasserstoff, Methyl, Ethyl, n-Propyl, iso-Propyl, Methoxy, Ethoxy, n-Propoxy und iso-Propoxy steht und $R^2$ und $R^3$ Wasserstoff, Methyl und Ethyl bedeuten.

2. Verfahren zur Herstellung der Verbindungen nach Anspruch 1, dadurch gekennzeichnet, daß

a) Isopren und ein Dienophil der Formel

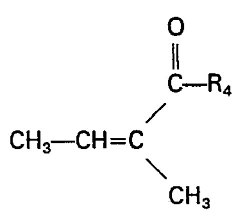

wobei $R_4$ Wasserstoff oder eine Alkylgruppe mit 2—3 Kohlenstoffatomen bedeutet, einer 1,4-Cycloaddition unterworfen werden und

b) gegebenenfalls das Reaktionsprodukt gemäß a)

b1) mittels Natriumborhydrid, Lithiumaluminiumhydrid oder Aluminiumisopropylat in den primären bzw. sekundären Alkohol,

b2) mittels Alkylmagnesiumhalogenid in den tertiären Alkohol, oder

b3) durch Oxidation zur Säure und anschließende Veresterung in den Ester, übergeführt wird.

3. Verwendung der Verbindung nach Anspruch 1 als Riechstoffe oder als Bestandteil von Riechstoffmischungen.

**Revendications**

1. Composés de formule générale

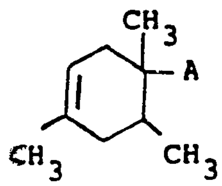

où A représente le groupe

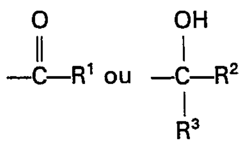

et $R^1$ est un hydrogène, le radical méthyle, éthyle, n-propyle, isopropyle, méthoxy, éthoxy, n-propoxy ou isopropoxy, et $R^2$ et $R^3$ représentent chacun un hydrogène ou le radical méthyle ou éthyle.

2. Procédé de préparation des composés selon la revendication 1, caractérisé en ce que

a) on soumet de l'isoprène et un diénophile de formule

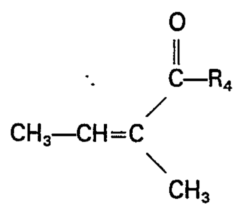

dans laquelle $R_4$ est un hydrogène ou un groupe alkyle ayant 1—3 atomes de carbone, à une cycloaddition sur les positions 1—4, et

b) on convertit éventuellement le produit de la réaction selon a)

b1) à l'aide de borohydrure de sodium, d'hydrure de lithium et d'aluminium ou d'isopropylate d'aluminium, en l'alcool primaire ou secondaire,

b2) à l'aide d'un halogénure d'alkylmagnésium, en l'alcool tertiaire, ou

b3) par oxydation pour donner l'acide et estérification, en l'ester.

3. Utilisation des composés selon la revendication 1 comme matières odorantes ou comme constituants de mélanges de matières odorantes.

**Claims**

1. Compounds of the general formula

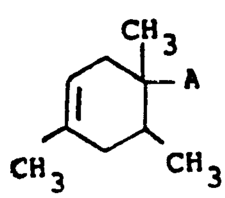

where A denotes the

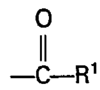 or the 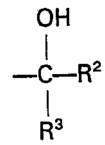

group
and where

$R^1$ represents hydrogen, methyl, ethyl, n-propyl, isopropyl, methoxy, ethoxy, n-propoxy and isopropoxy and $R^2$ denote hydrogen, methyl and ethyl.

2. Process for the preparation of the compounds according to claim 1, characterized in that

a) isoprene and a dienophile of the formula

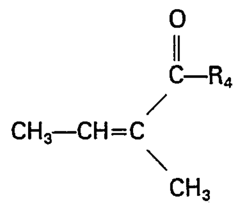

where $R_4$ denotes hydrogen or an alkyl group having 2—3 carbon atoms, are subjected to 1,4-cycloaddition and

b) if appropriate the reaction product according to a),

b1) is converted into the primary or secondary alcohol by means of sodium borohydride, lithium aluminium hydride or aluminium isopropylate,

b2) is converted into the tertiary alcohol by means of alkylmagnesium halide or

b3) is converted into the ester by oxidation to the acid and subsequent esterification.

3. Use of the compounds according to claim 1 as perfumes or as a constituent of perfume mixtures.